# EUROPEAN PATENT SPECIFICATION

(11) **EP 2 468 708 B1**
(45) Date of publication and mention of the grant of the patent: **09.11.2016**
(21) Application number: 10194330.6
(22) Date of filing: 09.12.2010
(51) Int. Cl.: C07C 45/48, C07C 225/22, C07C 49/76, C07C 49/813, C07C 49/83, C07C 49/784, C07C 49/794, C07C 49/84, C07D 307/46, C07D 333/22

(54) **Catalytic decarboxylative cross-ketonisation of aryl- and alkylcarboxylic acids using iron catalysts**
Katalysator-Decarboxyl-Kreuz-Ketonisierung von Aryl- und Alkylcarboxyl-Säuren mit Eisen-Katalysatoren
Kétonisation croisée catalytique décarboxylative d'acides aryl- et alkylecarboxyliques utilisant des catalyseurs ferreux

(43) Date of publication of application: 27.06.2012
(73) Proprietor: Cognis IP Management GmbH, 40589 Dusseldorf (DE)
(72) Inventor: Gooßen, Lukas J., 67663 Kaiserslautern (DE); Mamone, Patrizia, 67269 Gruenstadt (DE); Christoph, Oppel, 67661 Kaiserslautern (DE)
(74) Representative: BASF IP Association

(56) References cited:
- GRANITO, CHARLES ET AL: "Decarboxylation studies. II. Preparation of alkyl phenyl ketones", JOURNAL OF ORGANIC CHEMISTRY , 28, 879-81 CODEN: JOCEAH; ISSN: 0022-3263, 1963, XP002620841,
- WANG SHUQING ET AL: "Study on synthesis of m-chloropropiophenone from m-chlorobenzoic acid and propionic acid", JINGXI SHIYOU HUAGONG = SPECIALTY PETROCHEMICALS, ZHONGGUO SHIYOU HUAGONG ZONGGONGSI, CN, no. 4, 1 January 2005 (2005-01-01), pages 1-3, XP008132676, ISSN: 1003-9384
- DATABASE WPI Week 201067 Thomson Scientific, London, GB; AN 2010-L41304 XP002620866, & CN 101 805 251 A (UNIV NANTONG) 18 August 2010 (2010-08-18)
- PESTMAN R ET AL: "Reactions of Carboxylic Acids on Oxides - 2. Bimolecular Reaction of Aliphatic Acids to Ketones", JOURNAL OF CATALYSIS, ACADEMIC PRESS, DULUTH, MN, US, vol. 168, no. 2, 1 June 1997 (1997-06-01), pages 265-272, XP004459599, ISSN: 0021-9517, DOI: DOI:10.1006/JCAT.1997.1624
- CRESSELY J ET AL: "Evolution des especes carboxylates dans le cadre des syntheses CO-H2. Reduction de l'acide acetique sur systeme Co, Cu, Fe", MATERIALS CHEMISTRY AND PHYSICS, ELSEVIER, SWITZERLAND, TAIWAN, REPUBLIC OF CHINA, vol. 11, no. 5, 1 November 1984 (1984-11-01), pages 413-431, XP024148428, ISSN: 0254-0584, DOI: DOI:10.1016/0254-0584(84)90065-8 [retrieved on 1984-11-01]
- MAILHE M A: "Sur les catalyseurs des acides", BULLETIN DE LA SOCIETE CHIMIQUE DE FRANCE, SOCIETE FRANCAISE DE CHIMIE. PARIS, FRANCE, vol. 4, no. 15, 1 January 1914 (1914-01-01), pages 321-326, XP008132613, ISSN: 0037-8968
- Michael Renz: "Ketonization of Carboxylic Acids by Decarboxylation: Mechanism and Scope", European Journal of Organic Chemistry, vol. 2005, no. 6, 1 March 2005 (2005-03-01), pages 979-988, XP055076312, ISSN: 1434-193X, DOI: 10.1002/ejoc.200400546

## Description

The present invention pertains to a method to prepare aryl- and alkylcarboxylic acids using Fe-catalysts.

Aryl alkyl ketones are among the most important synthons in the industrial manufacture of commodities, fine chemicals, and pharmaceuticals. Simple derivatives, at early stages of the chemical value creation chain, are synthesised almost exclusively via Friedel-Crafts acylation of arenes, because the low cost of this reaction is unrivalled to date. However, two main issues are associated with this approach. Its low regioselectivity leads to the co-manufacture of large amounts of isomeric byproducts, and the use of acyl chlorides as starting materials activated with super-stoichiometric amounts of salt mediators (e.g., AlCl₃) is responsible for the formation of enormous quantities of salt waste.

Regioselective and less waste-intensive acylations, e.g., cross-couplings of sensitive aryl metal reagents with either preformed or in situ-generated carboxylic acid derivatives are widely used to access higher-value synthetic intermediates. However, their high cost precludes their application in the manufacture of base chemicals. This is also the case for alternative approaches, such as Heck-type reactions, or the Pd-catalysed decarboxylative cross-coupling of alpha-oxocarboxylate salts with aryl halides.

The production of asymmetrical ketones in the presence of iron or iron II is described in Granito, Charles et al. in "Decarboxylation Studies. II. Preparation of alkyl phenyl ketones", Journal of Organic Chemistry, 28, 879-81; Wang Shuqing et al. in "Study on synthesis of m-chloropropiophenone from m-chlorobenzoic acid and propionic acid", Jingxi Shiyou Huagong Zonggongsi, CN, no. 4, 1 January 2005, pages 1-3 and Database WPI Week 201067 Thomsen Scientific, London GB; AN 2010-L41304.

Pestmann R. et al. in "Reactions of carboxylic acids on Oxides - 2. Bimolecular reaction of Aliphatic Acids to Ketones", Journal of Catalysis, Academic Press, Duluth, MN, US, Vol. 168, no. 2, 1 June 1997, pages 265-272 and Cressely J. et al. in "Evolution des especes carboxylates dans le cadre des syntheses CO-H2. Reduction de l'acide acetique sur systeme Co, Cu, Fe", Materials Chemistry and Physics, Elsevier, Switzerland, Taiwan, Republic of China, vol. 11, no. 5, 1 November 1985, pages 413-431 describe the production of symmetrical ketones using magnetite. Mailhe M. A. in "Sur les catalyseurs des acides", Bulletin de la Societe Chimique de France, Societe Francaise de Chimie, Paris, France, vol. 4, no. 15. 1 January 1914, pages 321-326 describe alkyl aryl ketones in the gas phase by action of ferric oxide. Michael Renz in "Ketonization of Carboxylic Acids by Decarboxylation: Mechanism and Scope", European Journal of Organic Chemistry, vol. 2005, no. 6, 1 March 2005, pages 979-988 analyses ketonisation of carboxylic acids by decarboxylation.

Thus, a need remained for a broadly applicable, regioselective and waste-minimised aryl alkyl ketone synthesis starting from simple, inexpensive, and widely available chemicals.

As a solution to this long-standing problem, a catalytic process in which two different carboxylic acids are cross-coupled under release of CO₂ and water, to selectively give the aryl alkyl ketones.

The present invention relates to a method for the preparation of alkylarylketones according to the general formula (I) 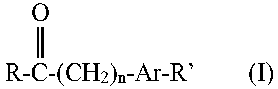 wherein R stands for a saturated or unsaturated, branched or linear alkyl or alkenyl moiety with 1 to 22 C-atoms, or an aromatic or hetero aromatic moiety with at least 5 C-atoms; Ar stands for an aromatic moiety, and R' represents a hydrogen atom, or a saturated or unsaturated, branched or linear alkyl or alkenyl moiety with 1 to 22 C-atoms, and n is Zero or a number of 1 to 4; comprising the steps
i) providing a blend of
   (a) an aromatic monocarboxylic acid
   (b) a second monocarboxylic acidselected from a saturated or unsaturated C8-C22 fatty acid, a branched or alicyclic monocarboxylic acid without heteroatoms or an alicyclic monocarboxylic acids containing heteroatoms for example oxygen or sulfur atoms, an aryl alkyl monocarboxylic acid as hydroxyphenyl alkyl monocarboxylic acid, alkoxyphenyl alkyl monocarboxylic acid, halogenphenyl alkyl monocarboxylic acid, a polycyclic monocarboxylic acid as naphthyl alkyl monocarboxylic acid.
      or mixtures thereof, and
   (c) an iron containing catalyst, and
   (d) a non-aqueous solvent;
ii) heating said blend to a temperature of between 250 and 380 °C, more preferably between 270 and 340 °C for at least 10 h and continuously remove water and CO₂ from the blend during the reaction takes place,
iii) after the reaction is terminated, the blend is distilled under reduced pressure and the reaction product is obtained in the distillate;
whereby the catalyst (c) is a mixed FE²⁺/Fe³⁺ oxide.

In a decarboxylative cross-ketonisation according to the present invention, the carboxylate groups would predefine the position of bond formation, potentially allowing a selective synthesis of any desired regioisomer. If mediated by catalytic amounts of an inexpensive metal with high selectivity for hetero-over homocoupling, the overall process would be advantageous both from economical and ecological standpoints. The decarboxylative homoketonisation of aliphatic carboxylic acids is an established strategy for the preparation of symmetrical dialkyl ketones or cyclic alkanones.

The selection of a proper catalyst (c) is the main feature of the present invention. First, it must be a catalyst, irrespectively of its physical form (solid, liquid) containing a mixed Fe²⁺/Fe³⁺ oxide.

This catalyst should be preferably used in solid form, for example as a powder. It is also beneficial to select those catalyst powders, having an average particle size of 1 - 500 nm, preferably with an average particle size of 1 - 50 nm and most preferable from 1 to 5 nm.

An example for such a mixed Fe-oxide is magnetite. Magnetite is a ferrimagnetic mineral with chemical formula Fe₃O₄, one of several iron oxides and a member of the spinel group. The chemical IUPAC name is iron(II,III) oxide and the common chemical name ferrous-ferric oxide. The formula for magnetite may also be written as FeO·Fe₂O₃, which is one part wüstite (FeO) and one part hematite (Fe₂O₃). This refers to the different oxidation states of the iron in one structure, not a solid solution. A useful catalyst is a commercially available PVP stabilized magnetite nanopwoder with an average particle size of 25 - 40 nm. The catalyst is used preferably in amounts from 5 mol% Fe²⁺ to 15 mol% Fe²⁺, calculated on the mols of all carboxylic acids (a) and (b) in the reaction blend.

The aromatic monocarboxylic acid (a) is one substrate for the method of the present invention. It is essential that this acid contains at least one aromatic part or moiety. Various known aromatic monocarboxylic acids are suitable in the method of the present invention. The term aromatic will also include heterocyclic aromatic substrates. Compound (a) is preferably selected from the group benzene monocarboxylic acid, toluene monocarboxylic acid, halogen benzene monocarboxylic acids, halogen toluene monocarboxylic acids, alkoxy benzene monocarboxylic acid, dialkylamino benzene monocarboxylic acid, hydroxy benzene monocarboxylic acid, trifluoromethane benzene monocarboxylic acid or heteroaromatic monocarboxylic acids as furanyl monocarboxylic acid or thiophenyl monocarboxylic acid or polycyclic monocarboxylic acid as naphthalene monocarboxylic acid or any mixtures of these acids.

The second monocarboxylic acid (b) is a saturated or unsaturated C8-C22 fatty acid, a branched or alicyclic monocarboxylic acid without heteroatoms or an alicyclic monocarboxylic acids containing heteroatoms for example oxygen or sulfur atoms, an aryl alkyl monocarboxylic acid as hydroxyphenyl alkyl monocarboxylic acid, alkoxyphenyl alkyl monocarboxylic acid, halogenphenyl alkyl monocarboxylic acid, a polycyclic monocarboxylic acid as naphthyl alkyl monocarboxylic acid or a mixture thereof.

For the sake of it is noted that the benzylic acid (b) must be structurally different to aromatic acid (a).

It was found that good results will be achieved if the mol ratio of (a) to (b) is in the range from 1 : 2 to 2 : 1. However, if almost equimolar mixtures of aromatic and aliphatic carboxylic acids are subjected to the above catalysts, dialkyl and aryl alkyl ketones are obtained at least in a 1: 9 ratio. An especially preferred ratio is in the range from 1.5 : 1 to 1.1 : 1 and particularly 1.2 : 1.

The presence of a non-aqueous solvent (d) is also compelling. Examples of proper solvents are sulfolane, tetraglyme, N-methyl-2-pyrrolidone or a eutectic mixture of diphenylether and biphenyl (Dowtherm^{®}A). Other suitable solvents are selected from quinoline, 1,3-dimethyl-3,4,5,6-tetrahydro-2(1H)-pyrimidinone trichlorobenzene, 1,4-butandiole, tetradecane, mineral oil, naphthalene or an eutectic mixture of diphenylether and biphenyl. Mixtures of the above mentioned solvents are also suitable.

The reaction is carried out at elevated temperatures, which is 200 °C and higher. The temperature during the reaction step ii) is kept between 250 and 380 °C, more preferably between 270 and 340 °C.

Due to the probably detrimental effect of oxygen it is preferred to carry out the reaction under oxygen-free conditions, which means to apply a N₂ or Ar-atmosphere.

### Examples

### Synthesis of 2-phenyl-1-(3-tolyl)-ethanone

An oven-dried Schlenk flask was charged with magnetite nanopowder (< 50 nm, 590 mg, 98% purity, 2.50 mmol), 3-toluic acid (8.17 g, 60.0 mmol), phenylacetic acid (6.88 g, 99% purity, 50.0 mmol), and Dowtherm® A (100 mL).

The apparatus was evacuated and flushed with nitrogen three times. The reaction mixture was heated to reflux (250 °C) overnight in a metal bath, while continuously removing CO₂ and water in a slow stream of nitrogen. The mixture was then distilled trap-to-trap (60-110 °C, 10-3 mbar) to give a clear, yellow distillate and dark brown residue. Compound 3aa was separated from Dowtherm^{®} A

A by fractional distillation over a Vigreux column (Dowtherm^{®} A: 55-60 °C, 10-3 mbar) and obtained as a pale yellow oil that solidified to an off-white solid upon cooling (100-105 °C, 10-3 mbar, 8.43 g, 80%, as a 10:1 mixture with 1,3-diphenylacetone).

For spectroscopic characterization, the product was further purified by column chromatography (SiO₂, ethyl acetate/hexane gradient). The spectroscopic data matched those reported in the literature for 2-phenyl-1-(3-tolyl)-ethanone (CAS 95606-81-8) [compound (3aa) in table 1].

Further examples with different aromatic carboxylic acids where conducted. The different products are shown in the following table 1:

**Table 1**

| | | | |
|---|---|---|---|
| 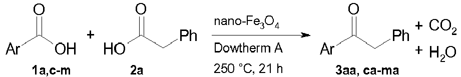 | | | |

| product | yield [%] | product | yield [%] |
|---|---|---|---|
| 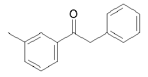 | 80 | 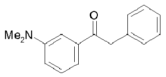 | 43 |
| **3aa** | | **3ha** | |
| 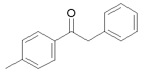 | 65 | 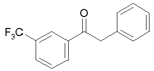 | 83 |
| **3ca** | | **3ia** | |
| 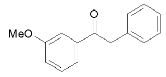 | 69 | 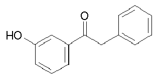 | 27 |
| **3da** | | **3ja** | |
| 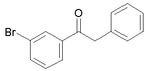 | 69 | 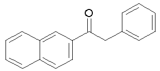 | 80 |
| **3ea** | | **3ka** | |
| 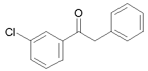 | 83 | 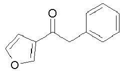 | 32 |
| **3fa** | | **3la** | |
| 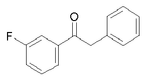 | 78 | 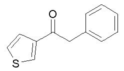 | 64^{[a]} |
| **3ga** | | **3ma** | |

| | | | |
|---|---|---|---|
| *Conditions:* 1.20 mmol **1**, 1.00 mmol **2a,** 0.05 mmol Fe₃O₄, 21 h, 250 °C, 2 mL Dowtherm A, isolated yields. [a] 0.07 mmol Fe₃O₄. | | | |

According to the procedure as described in connection with compound 3aa reaction between aromatic monocarboxylic acids (in particular 3-methylbezene monocarboxylic acid) with different non aromatic acids of type (b) were conducted.

The results are shown in the table 2:

**Table 2:**

| | | | |
|---|---|---|---|
| 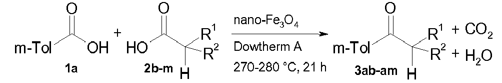 | | | |

| product | yield [%] | product | yield [%] |
|---|---|---|---|
| 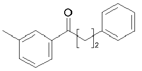 | 78 | 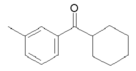 | 53^{[a]} |
| **3ab** | | **3ah** | |
| 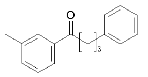 | 80 | 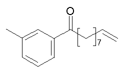 | 51^{[b]} |
| **3ac** | | **3ai** | |
| 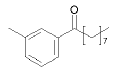 | 86 | 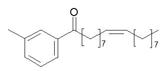 | 70 |
| **3ad** | | **3aj** | |
| 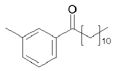 | 85 | 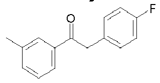 | 76^{[c]} |
| **3ae** | | **3ak** | |
| 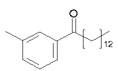 | 76 | 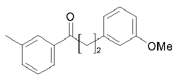 | 59 |
| **3af** | | **3al** | |
| 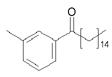 | 74 | 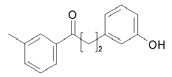 | 41 |
| **3ag** | | **3am** | |

| | | | |
|---|---|---|---|
| *Conditions:* 1.20 mmol **1a,** 1.00 mmol **2**, 0.07 mmol Fe₃O₄, 280 °C, 21 h, 2 mL Dowtherm A, isolated yields. [a] 72 h. [b] Mixture of isomers. [c] 270 °C. | | | |

## Claims

1. A method for the preparation of alkylarylketones according to the general formula (I) 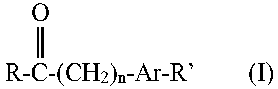 wherein R stands for a saturated or unsaturated, branched or linear alkyl or alkenyl moiety with 1 to 22 C-atoms, or an aromatic or hetero aromatic moiety with at least 5 C-atoms; Ar stands for an aromatic moiety, and R' represents a hydrogen atom, or a saturated or unsaturated, branched or linear alkyl or alkenyl moiety with 1 to 22 C-atoms, and n is zero or a number of 1 to 4; comprising the steps
i) providing a blend of
(a) an aromatic monocarboxylic acid
(b) a second monocarboxylic acid, selected from a saturated or unsaturated C8-C22 fatty acid, a branched or alicyclic monocarboxylic acid without heteroatoms or an alicyclic monocarboxylic acids containing heteroatoms for example oxygen or sulfur atoms, an aryl alkyl monocarboxylic acid as hydroxyphenyl alkyl monocarboxylic acid, alkoxyphenyl alkyl monocarboxylic acid, halogenphenyl alkyl monocarboxylic acid, a polycyclic monocarboxylic acid as naphthyl alkyl monocarboxylic acid, or mixtures thereof, and
(c) an iron containing catalyst, and
(d) a non-aqueous solvent;
ii) heating said blend to a temperature of between 250 and 380 °C, preferably between 270 and 340 °C for at least 10 h and continuously remove water and CO₂ from the blend during the reaction takes place,
iii) after the reaction is terminated, the blend is distilled under reduced pressure and the reaction product is obtained in the distillate;
**characterized in that** the catalyst (c) is a mixed Fe^{2+/}Fe³⁺ oxide.

2. A method according to claim 1, **characterized in that** the catalyst (c) is used in the form of a powder with an average particle size in the range of 1 - 500 nm, preferably with an average particle size of 1 - 50 nm, and preferably 1 - 5 nm.

3. A method according to at least one of the preceding claims **characterized in that** the catalyst (c) is used in amounts from 5 mol% Fe²⁺ to 15 mol% Fe²⁺, calculated on the mols of all carboxylic acids (a) and (b) in the reaction blend.

4. A method, according to at least one of the preceding claims, **characterized in that** the monocarboxylic acid (a) is selected from the group benzene monocarboxylic acid, toluene monocarboxylic acid, halogen benzene monocarboxylic acids, halogen toluene monocarboxylic acids, alkoxy benzene monocarboxylic acid, dialkylamino benzene monocarboxylic acid, hydroxy benzene monocarboxylic acid, trifluoromethane benzene monocarboxylic acid or heteroaromatic monocarboxylic acids as furanyl monocarboxylic acid or thiophenyl monocarboxylic acid or polycyclic monocarboxylic acid as naphthalene monocarboxylic acid.

5. A method according to at least one of the preceding claims, **characterized in that** the non-aqueous solvent is selected from sulfolane, tetraglyme, n-methyl-2-pyrrolidone, quinoline, 1,3-dimethyl-3,4,5,6-tetrahydro-2(1H)-pyrimidinone trichlorobenzene, 1,4-butandiole, tetradecane, mineral oil, naphthalene or an eutectic mixture of diphenylether and biphenyl.

6. A method according to at least one of the preceding claims, **characterized in that** the reaction step (ii) is carried out for a period of time of 10 h to 24 h.

7. A method according to at least one of the preceding claims, **characterized in that** the reaction is carried out under a N₂ or Ar-atmosphere.

8. A method according to at least one of the preceding claims **characterized in that** the mol ratio of (a) to (b) is in the range from 1 : 2 to 2 : 1, preferred is a ratio of 1.2 : 1.

## Patentansprüche

1. Verfahren zur Herstellung von Alkylarylketonen gemäß der allgemeinen Formel (I) 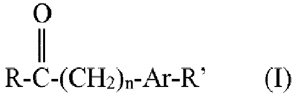 worin R für eine gesättigte oder ungesättigte, verzweigte oder lineare Alkyl- oder Alkenylgruppe mit 1 bis 22 C-Atomen oder eine aromatische oder heteroaromatische Gruppe mit mindestens 5 C-Atomen steht; Ar für eine aromatische Gruppe steht und R' für ein Wasserstoffatom oder eine gesättigte oder ungesättigte, verzweigte oder lineare Alkyl- oder Alkenylgruppe mit 1 bis 22 C-Atomen steht und n für null oder eine Zahl von 1 bis 4 steht; bei dem man
i) eine Mischung von
(a) einer aromatischen Monocarbonsäure,
(b) einer zweiten Monocarbonsäure, die aus einer gesättigten oder ungesättigten C8-C22-Fettsäure, einer verzweigten oder alicyclischen Monocarbonsäure ohne Heteroatome oder einer alicyclischen Monocarbonsäure mit Heteroatomen, beispielsweise Sauerstoff- oder Schwefelatomen, einer Arylalkylmonocarbonsäure wie Hydroxyphenylalkylmonocarbonsäure, Alkoxyphenylalkylmonocarbonsäure, Halogen-phenylalkylmonocarbonsäure, einer polycyclischen Monocarbonsäure wie Naphthylalkylmonocarbonsäure oder Mischungen davon ausgewählt wird, und
(c) einem eisenhaltigen Katalysator und
(d) einem nichtwässrige Lösungsmittel bereitstellt;
ii) die Mischung mindestens 10 h auf eine Temperatur zwischen 250 und 380°C, vorzugsweise zwischen 270 und 340°C erhitzt und während des Ablaufs der Reaktion kontinuierlich Wasser und CO₂ aus der Mischung entfernt,
iii) die Mischung nach Beendigung der Reaktion unter vermindertem Druck destilliert und das Reaktionsprodukt im Destillat erhält;
**dadurch gekennzeichnet, dass** es sich bei dem Katalysator (C) um ein Fe²⁺/Fe³⁺-Mischoxid handelt.

2. Verfahren nach Anspruch 1, **dadurch gekennzeichnet, dass** der Katalysator (c) in Form eines Pulvers mit einer durchschnittlichen Teilchengröße im Bereich von 1-500 nm, vorzugsweise mit einer durchschnittlichen Teilchengröße von 1-50 nm und vorzugsweise 1-5 nm verwendet wird.

3. Verfahren nach mindestens einem der vorhergehenden Ansprüche, **dadurch gekennzeichnet, dass** der Katalysator (c) in Mengen von 5-Mol-% Fe²⁺ bis 15 Mol-% Fe²⁺, berechnet auf die Mole aller Carbonsäuren (a) und (b) in der Reaktionsmischung, verwendet wird.

4. Verfahren nach mindestens einem der vorhergehenden Ansprüche, **dadurch gekennzeichnet, dass** die Monocarbonsäure (a) aus der Gruppe Benzolmonocarbonsäure, Toluolmonocarbonsäure, Halogenbenzolmonocarbonsäuren, Halogentoluolmonocarbonsäuren, Alkoxybenzolmonocarbonsäure, Dialkylaminobenzolmonocarbonsäure, Hydroxybenzolmonocarbonsäure, Trifluormethanbenzolmonocarbonsäure oder heteroaromatische Monocarbonsäuren wie Furanylmonocarbonsäure oder Thiophenylmonocarbonsäure oder polycyclische Monocarbonsäure wie Naphthalinmonocarbonsäure ausgewählt wird.

5. Verfahren nach mindestens einem der vorhergehenden Ansprüche, **dadurch gekennzeichnet, dass** das nichtwässrige Lösungsmittel aus Sulfolan, Tetraglyme, N-Methyl-2-pyrrolidon, Chinolin, 1,3-Dimethyl-3,4,5,6-tetrahydro-2(1H)-pyrimidinon, Trichlorbenzol, 1,4-Butandiol, Tetradecan, Mineralöl, Naphthalin oder einer eutektischen Mischung von Diphenylether und Biphenyl ausgewählt wird.

6. Verfahren nach mindestens einem der vorhergehenden Ansprüche, **dadurch gekennzeichnet, dass** der Reaktionsschritt (ii) über einen Zeitraum von 10 h bis 24 h durchgeführt wird.

7. Verfahren nach mindestens einem der vorhergehenden Ansprüche, **dadurch gekennzeichnet, dass** die Reaktion unter N₂- oder Argonatmosphäre durchgeführt wird.

8. Verfahren nach mindestens einem der vorhergehenden Ansprüche, **dadurch gekennzeichnet, dass** das Molverhältnis von (a) zu (b) im Bereich von 1:2 bis 2:1 liegt und ein Verhältnis von 1,2:1 bevorzugt ist.

## Revendications

1. Procédé de préparation d'alkylarylcétones selon la formule générale (I) 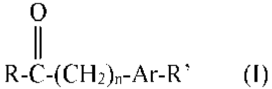 dans laquelle R désigne un fragment alkyle ou alcényle saturé ou insaturé, ramifié ou linéaire avec 1 à 22 atomes C, ou un fragment aromatique ou hétéroaromatique avec au moins 5 atomes C ; Ar désigne un fragment aromatique, et R' représente un atome d'hydrogène, ou un fragment alkyle ou alcényle saturé ou insaturé, ramifié ou linéaire avec 1 à 22 atomes C, et n est zéro ou un nombre de 1 à 4 ; comprenant les étapes de
i) fourniture d'un mélange de
(a) un acide monocarboxylique aromatique
(b) un deuxième acide monocarboxylique, choisi parmi un acide gras en C₈-C₂₂ saturé ou insaturé, un acide monocarboxylique ramifié ou alicyclique sans hétéroatomes ou des acides monocarboxyliques alicycliques contenant des hétéroatomes, par exemple, des atomes d'oxygène ou de soufre, un acide arylalkylmonocarboxylique tel qu'un acide hydroxyphénylalkylmonocarboxylique, un acide alcoxyphénylalkylmonocarboxylique, un acide halogénophénylalkylmonocarboxylique, un acide monocarboxylique polycyclique tel qu'un acide naphtylalkylmonocarboxylique, ou des mélanges de ceux-ci , et
(c) un catalyseur contenant du fer, et
(d) un solvant non aqueux ;
ii) chauffage dudit mélange à une température comprise entre 250 et 380 °C, de préférence entre 270 et 340 °C pendant au moins 10 h et élimination continue de l'eau et de CO₂ à partir du mélange pendant que la réaction se produit,
iii) une fois que la réaction est terminée, le mélange est distillé sous pression réduite et le produit de réaction est obtenu dans le distillat ;
**caractérisé en ce que** le catalyseur (c) est un oxyde mixte Fe²⁺/Fe³⁺.

2. Procédé selon la revendication 1, **caractérisé en ce que** le catalyseur (c) est utilisé sous la forme d'une poudre ayant une taille de particule moyenne dans la plage de 1 à 500 nm, de préférence ayant une taille de particule moyenne de 1 à 50 nm, et de préférence 1 à 5 nm.

3. Procédé selon au moins une des revendications précédentes **caractérisé en ce que** le catalyseur (c) est utilisé dans des quantités de 5 % en moles de Fe²⁺ à 15 % en moles de Fe²⁺, calculées sur les moles de l'ensemble des acides carboxyliques (a) et (b) dans le mélange de réaction.

4. Procédé, selon au moins une des revendications précédentes, **caractérisé en ce que** l'acide monocarboxylique (a) est choisi dans le groupe de l'acide benzènemonocarboxylique, l'acide toluènemonocarboxylique, des acides halogénobenzènemonocarboxyliques, des acides halogénotoluènemonocarboxyliques, un acide alcoxybenzènemonocarboxylique, un acide dialkylaminobenzènemonocarboxylique, l'acide hydroxybenzènemonocarboxylique, l'acide trifluorométhanebenzènemonocarboxylique ou des acides monocarboxyliques hétéroaromatiques tels que l'acide furanylmonocarboxylique ou l'acide thiophénylmonocarboxylique ou un acide monocarboxylique polycyclique tel que l'acide naphtalènemonocarboxylique.

5. Procédé selon au moins une des revendications précédentes, **caractérisé en ce que** le solvant non aqueux est choisi parmi le sulfolane, le tétraglyme, la n-méthyl-2-pyrrolidone, la quinoléine, le 1,3-diméthyl-3,4,5,6-tétrahydro-2(1H)-pyrimidinonetrichlorobenzène, le 1,4-butanediole, le tétradécane, l'huile minérale, le naphtalène ou un mélange eutectique d'éther diphénylique et de biphényle.

6. Procédé selon au moins une des revendications précédentes, **caractérisé en ce que** l'étape de réaction (ii) est conduite pendant une durée de 10 h à 24 h.

7. Procédé selon au moins une des revendications précédentes, **caractérisé en ce que** la réaction est conduite sous une atmosphère de N₂ ou Ar.

8. Procédé selon au moins une des revendications précédentes **caractérisé en ce que** le rapport molaire de (a) à (b) est dans la plage de 1:2 à 2:1, de préférence un rapport de 1,2:1.
